# EUROPEAN PATENT SPECIFICATION

(11) **EP 4 240 435 B1**
(45) Date of publication and mention of the grant of the patent: **25.09.2024**
(21) Application number: 21888837.8
(22) Date of filing: 04.11.2021
(51) Int. Cl.: A61L 27/24, A61L 27/26, A61L 15/32, A61L 27/12, A61L 27/20, A61L 27/42, A61L 27/50, A61L 27/14, A61L 27/02, A61L 27/54

(54) **MULTI-LAYERED COLLAGEN STRUCTURE**
MEHRSCHICHTIGE KOLLAGENSTRUKTUR
STRUCTURE DE COLLAGÈNE MULTICOUCHE

(30) Priority: 05.11.2020 US 202063109888 P
(43) Date of publication of application: 13.09.2023
(73) Proprietor: Datum Dental Ltd., Lod 7120101 (IL)
(72) Inventor: KAPRI, Ran, 7680400 Mazkeret Batya (IL); BAYER, Thomas, 6248310 Tel Aviv (IL); GOLDLUST, Arie, 7404606 Nes Ziona (IL)
(74) Representative: Pichova, Vanda
(86) International application number: PCT/IL2021/051315
(87) International publication number: WO 2022/097149

(56) References cited:
- CN-U- 212 308 516
- US-A1- 2009 232 875
- US-A1- 2014 105 954
- US-A1- 2014 105 954
- US-A1- 2016 199 537
- LEVINGSTONE TANYA J ET AL: "Multi-layered collagen-based scaffolds for osteochondral defect repair in rabbits", 8 May 2008 (2008-05-08), XP093143969, Retrieved from the Internet <URL:https://pdf.sciencedirectassets.com/271870/1-s2.0-S0142961208X00171/1-s2.0-S0142961208003542/main.pdf> [retrieved on 20240321]
- TAMPIERI, A. ; SANDRI, M. ; LANDI, E. ; PRESSATO, D. ; FRANCIOLI, S. ; QUARTO, R. ; MARTIN, I.: "Design of graded biomimetic osteochondral composite scaffolds", BIOMATERIALS, ELSEVIER, AMSTERDAM, NL, vol. 29, no. 26, 1 September 2008 (2008-09-01), AMSTERDAM, NL , pages 3539 - 3546, XP022822736, ISSN: 0142-9612, DOI: 10.1016/j.biomaterials.2008.05.008

## Description

### FIELD OF THE INVENTION

The invention is directed to a multilayered collagen structure having at least two layers having different densities. The invention is further directed to a method for preparing such multilayered structure, wherein the method comprises compressing fibrillated collagen in the presence of at least one distancing element.

### BACKGROUND OF THE INVENTION

Dental guided bone regeneration (GBR) and guided tissue regeneration (GTR) are known procedures for bone and tissue augmentation. Both GBR and GTR require the exclusion of unwanted tissues from occupying a space where the regenerated bone or tissue is to grow into. The secluded space should allow cells from bone origin, mesenchymal stem cells, gingival cells, fibroblasts and the like to proliferate, migrate and may differentiate into bone forming cells. This secluded space should be maintained for the new bone to mature and become stable with minimal resorption. It has been shown that the highest success rates of GBR procedures are achieved by a combination of bone grafts and barrier membranes that prevent undesired tissue from occupying the space into which the bone is regenerated.

In many instances, the bone envelop around dental implants is not ideal and does not support long term protection. Many times, peri-implant infections, which resemble periodontal disease around natural teeth, may develop. In other instances, the aesthetic outcome of the implant procedure will be compromised due to the reflection of the implant through the bone and/or soft tissue or due to recession of the soft tissue that exposes the metal components of the implant.

It is possible to augment oral soft tissues when necessary, e.g., for aesthetics, e.g., by harvesting autogenous dense connective tissue from the palate or the tuberosity. Allogenic grafts are also used widely with limited outcome. However, such procedures may require additional surgical procedures and may cause pain, infections or be rejected by the body. Therefore, there is a need in the art for a biocompatible and, possibly, at least biodegradable, device for augmenting soft and hard tissue around natural teeth and dental implants. Such a device should be prepared from a biocompatible and possibly biodegradable material.

One type of known biocompatible and biodegradable material is collagen. The collagen protein constitutes approximately 30% of the proteins in a living body and functions as a support for bone and cell adherence. Accordingly, collagen is known to be a useful biomaterial, used for example in cell culture substrates, as well as a scaffold material for regenerative medicine, including tissue engineering of cartilage, bone, ligaments, corneal stroma and skin. Collagen is used also as an implantation material, for example as a wound dressing material, bone grafting material, hemostatic material, or anti-adhesive material.

Thus, it would be desirable to prepare a collagen structure that is both biocompatible and is further capable of augmenting both soft and hard tissue, wherein each type of tissue remains in the region in which it is desired.

US 2014/105954 A1 discloses a multi-layer collagen scaffold, ideally crosslinked. It is further disclosed that a three-layer collagen scaffold is prepared, wherein: - the first outer layer comprises collagen, typically Type I collagen, and hydroxyapatite (HA); - the inner layer comprises a type I collagen, non-Type I Collagen (ideally Type II collagen), HA and, optionally, one or more GAG (glycosaminoglycan); and - the second outer layer comprises a composite of Type I collagen, non-Type I collagen (ideally Type II collagen) and optionally one or more GAGs and/or a biologic.

Levingstone Tanya J ET AL: "Multi-layered collagen-based scaffolds for osteochondral defect repair in rabbits", Royal College of Surgeons in Ireland, 8 May 2008, URL:https:// pdf.sciencedirectassets.com/271870/1-s2.0-50142961208X00171/1-s2.0-50142961208003542/ main.pdf discloses a multi-layer collagen scaffold, comprising bone, intermediate and cartilage layers. The bone layer contains 0.5% (w/v) microfibrillar bovine tendon type I collagen (Coll) and 1% (w/v) Hydroxyapatite (HA) powder; the intermediate layer contains Coll 0.5% (w/v), type II collagen (Col2) 0.5% (w/v) and HA 0.2% (w/v); and the cartilage layer consists of 0.125% (w/v) Coll, 0.375% (w/v) Col2 and 0.05% (w/v) HA.

US 2009/232875 A1 discloses a multilayer structure including a first upper layer consisting of an organic matrix comprising collagen and at least a lower layer consisting of a composite matrix including hydroxyapatite (HA) and collagen. The multi-layer structure is characterised by the presence of a collagen gradient: starting from an upper layer comprising 100% of collagen, the collagen content in each subsequent layers decreases until the lowest layer, in which the relative collagen content is less than 10%; and wherein the amount of hydroxyapatite is complementary to the amount of collagen (the combination of collagen and hydroxyapatite content results in 100% of the mass of a layer).

### SUMMARY OF THE INVENTION

The present invention provides a layered structure comprising at least two collagen layers according to claim 1.

In some embodiments, the present invention provides a layered structure comprising at least two collagen layers each having a different density, wherein said at least two collagen layers comprise at least one first collagen layer and at least one second collagen layer; wherein said at least one second collagen layer (low density layer) has a density of up to about 90% of the first collagen layer (high density layer).

In some embodiments, the present invention provides a layered structure comprising at least two collagen layers each having a different density, wherein said at least two collagen layers comprise at least two first collagen layers and at least one second collagen layer; wherein said at least one second collagen layer (inner layer) is positioned between each of said at least two first collagen layers (external layers).

In some embodiments, the present invention provides a layered structure comprising at least two collagen layers each having a different density, wherein said at least two collagen layers comprise at least two first collagen layers and at least one second collagen layer; wherein said at least one second collagen layer (inner layer) is positioned between each of said at least two first collagen layers (external layers), and wherein said at least one second collagen layer (low density layer) has a density of up to about 90% of the at least two first collagen layers each independently (high density layers).

In some embodiments, the present invention provides a layered structure comprising at least two collagen layers each having a different density, wherein said at least two collagen layers comprise at least two first collagen layers and at least one second collagen layer; wherein said at least one second collagen layer (inner layer) is positioned between each of said at least two first collagen layers (external layers), and wherein said at least two first collagen layers (low density layer) have each independently a density of up to about 90% of the at least one second collagen layer (high density layers).

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features and advantages thereof, may best be understood by reference to the following detailed description when read with the accompanied drawings. Embodiments of the invention are illustrated by way of example and not limitation in the figures of the accompanying drawings, in which like reference numerals indicate corresponding, analogous or similar elements, and in which:
Figure 1 presents a photographic image of a three-layered collagen structure according to embodiments of the invention.
Figure 2 presents a 2X magnification of a three-layered collagen structure according to embodiments of the invention.
Figure 3 presents a photomicrograph of an implant of a layered structure of the invention in a dog, four weeks after the implant, implanted for repairing an L-shaped body defect, wherein the collagen structure is and marked "M", showing the initiation of the multilayered collagen structure ossification.
Figure 4 presents a photomicrograph of an implant in a dog, 24 weeks after the implant, implanted for repairing an L-shaped body defect.
Figure 5 presents a photomicrograph of an implant in a dog, four weeks after the implant, implanted for repairing an L-shaped body defect, presenting two external layers and one internal layer of the multilayered collagen implanted structure.
Figures 6A and 6B present the preparation of a structure of the invention without using spacers (Fig. 6A) and with using spacers (Fig. 6B).

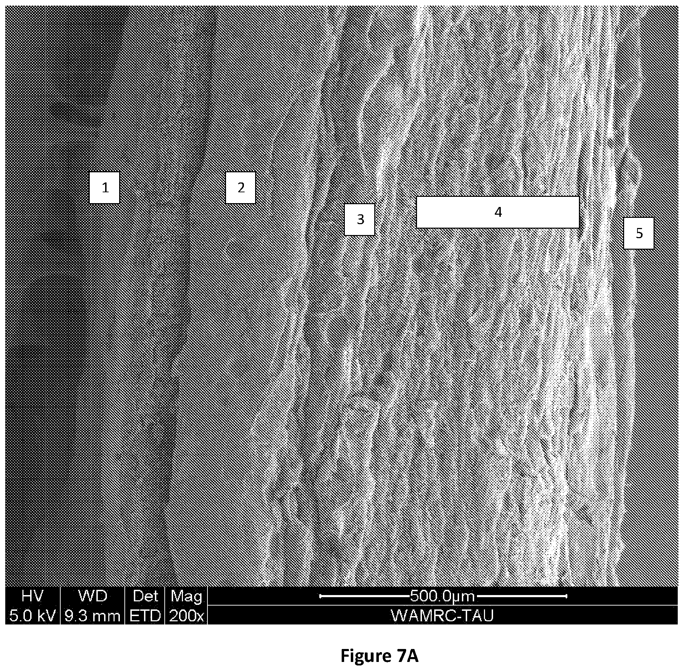

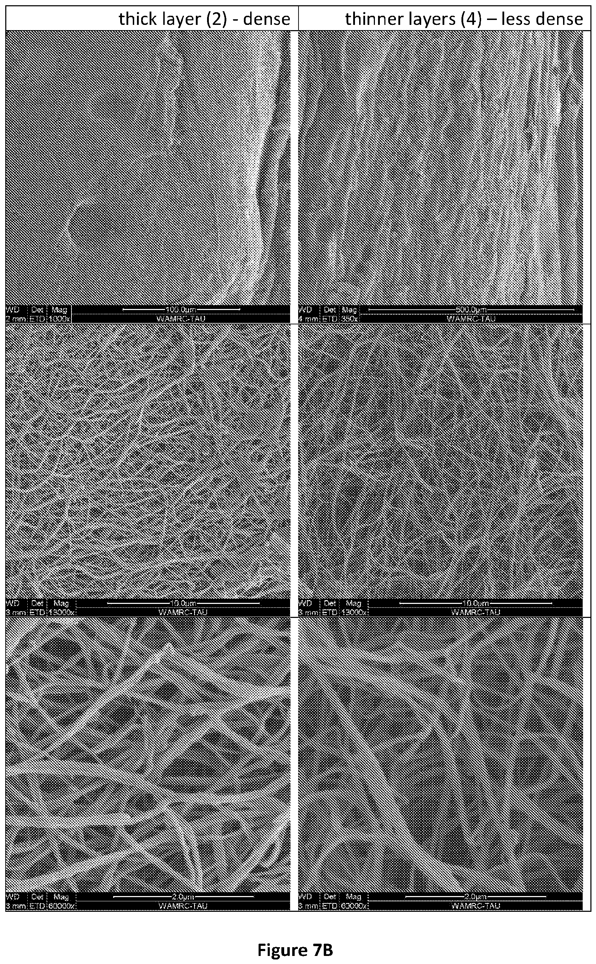

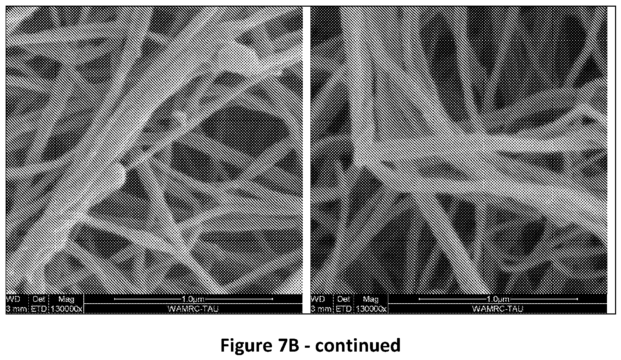

Figures 7A and 7B show a layered structure of the invention having 5 different density layers. Figure 7A shows the electron microscopic image (lower resolution): cross section of the multi-layered membrane #1, #5: denser outer layers of the multi-layered membrane; #2, #3, #4: inner, lower dense layers of the multi-layered membrane. Figure 7B shows the electron microscopic image (higher resolutions): comparison between lower and higher dense layers of the multi-layered membrane (denser and less dense appearance of collagen fibers).

### DETAILED DESCRIPTION OF EMBODIMENTS OF THE INVENTION

The invention provides a layered structure comprising at least two collagen layers each having a different density

The term "layered structure" should be understood to encompass an assembly of at least two layers of collagen being placed one on top of the other. The layered structure of the invention comprises at least two collagen layers, wherein each of these two layers has different density than the other. This term is interchangeably used with the term "collagen layered structure" and "collagen layered membrane". The layered structure can be part of a device, matrix or a scaffold.

Collagen is the main structural protein in the extracellular matrix in the various connective tissues in the body, consisting of amino acids bound together to form a triple helix of elongated fibril known as a collagen helix. It is mostly found in connective tissue and membranes such as cartilage, bones, tendons, ligaments, and skin.

Depending upon the degree of mineralization, collagen layers may have different densities. In some embodiments collagen layers may be rigid (such as in bone), compliant (such as in tendon), or have a gradient from rigid to compliant (such as in cartilage).

In some embodiments, said layered structure is a membrane. In other embodiments, said layered structure is an implantable membrane.

Soft and/or hard tissue may be augmented differently by each of the collagen layers of the layered structure of the invention, due to the difference in density of each collagen layer. According to some embodiments, the regenerated tissue may be maintained for more than 6 months.

According to some embodiments, the layered structure of the invention may be used as a surgical scaffold for soft tissue growth/augmentation, e.g., dorsal augmentation. According to further embodiments, the layered structure of the invention may be used for reinforcing soft tissues when necessary, e.g., during hernia repair. According to further embodiments, the layered structure of the invention may be used for treatments including bone growth/augmentation, e.g., guided bone regeneration (GBR) and guided tissue regeneration (GTR), such as in the treatment of periodontal defects. The layered structure of the invention may further be used for soft tissue growth/augmentation, such as gingival augmentation, face lifting/repairs, lifting/augmentation of soft tissue and cartilage, e.g., of the nose or ear. According to some embodiments, the layered structure of the invention may be used for the combined growth/augmentation of any soft and hard tissues, depending on the area in which the membrane is placed.

According to some embodiments, the layered structure of the invention may be used to prevent adhesions in tissues and organs. The prevention of adhesions may occur by the use of a layered structure of the invention comprising a relatively low-density internal collagen layer that may degrade at a higher rate than the external denser collagen layers, such that when the internal layer degrades the layered structure of the invention essentially separates into two separate membranes, thereby preventing adhesions between the tissue above the layered structure of the invention and below it.

According to some embodiments, the layered structure of the invention provides long term protection and aesthetics for teeth and dental implants and other augmented sites. As detailed herein, the implanted layered structure of the invention may be partially or fully populated, degraded and replaced by the surrounding tissue.

According to some embodiments, one or more of the layers of the layered structure of the invention provides promotion for the growth of various cell types. According to some embodiments, the promotion for various cell types is dependent on the density of the layer. Thus, specific areas of the layered structure of the invention promotes the growth of specific types of tissue, including gum tissue and bone tissue.

According to some embodiments, the growth of bone tissue is promoted in relatively dense collagen layers, while the growth of soft tissue is promoted in relatively less dense collagen layers.

According to some embodiments, the layered structure of the invention comprises relatively less dense collagen layers sandwiched between relatively highly dense collagen layers, such that the high-density collagen layers are exposed to the surrounding tissue. According to some embodiments, the high-density collagen layers promote the growth of bone tissue, while the low-density collagen layers promote the growth of soft tissue. According to some embodiments, fibroblasts may penetrate into internal collagen layer, e.g., through at least one high density collagen layer and/or under/over/around the layered structure of the invention, enabling the growth of soft tissue in the internal, low density, collagen layers.

In some embodiments, the terms "highly dense collagen layer", "relatively highly dense collagen layer", and the like, define layers having an average density of about 1.41g/cm³. Further, the terms "less dense collagen layer", "low density collagen layer", "relatively low-density collagen layer" and the like, define layers having an average density of about 1.17 g/cm³. Further, the term "about" includes a range that is ±10% of the disclosed value. According to some embodiments, the density of the relatively highly dense collagen layer is in the range of about 1.0-1.7 g/cm³. According to some embodiments, the density of the relatively highly dense collagen layer is in the range of about 1.0-2 g/cm³. According to some embodiments, the density of the relatively low-density collagen layer is in the range of about 0.4-1.0 g/cm³.

In some embodiments, the density is determined by Hg porosimetry.

According to some embodiments, the layered structure of the invention comprises at least two collagen layers, wherein at least one collagen layer has a relatively low density and at least one collagen layer having a relatively higher density, and wherein the relatively low-density collagen layer has a density of up to about 90% of the high density layer. According to some embodiments, the relatively low-density collagen layer has a density of up to about 85% of the high-density layer. According to some embodiments, the relatively low-density collagen layer has a density of up to about 80% of the high-density collagen layer. According to some embodiments, the relatively low-density collagen layer has a density of up to about 75% of the high-density collagen layer. According to some embodiments, the relatively low-density collagen layer has a density of up to about 70% of the high-density layer.

According to some embodiments, the relatively low-density collagen layer has a density of between 10% to 90% of the high-density collagen layer. According to some embodiments, the relatively low-density collagen layer has a density of between 20% to 90% of the high-density collagen layer. According to some embodiments, the relatively low-density collagen layer has a density of between 30% to 90% of the high-density collagen layer. According to some embodiments, the relatively low-density collagen layer has a density of between 40% to 90% of the high-density collagen layer. According to some embodiments, the relatively low-density collagen layer has a density of between 50% to 90% of the high-density collagen layer. According to some embodiments, the relatively low-density collagen layer has a density of between 60% to 90% of the high-density collagen layer. According to some embodiments, the relatively low-density collagen layer has a density of between 70% to 90% of the high-density collagen layer. According to some embodiments, the relatively low-density collagen layer has a density of between 80% to 90% of the high-density collagen layer.

In some embodiments, the present invention provides a layered structure comprising at least two collagen layers each having a different density, wherein said at least two collagen layers comprise at least one first collagen layer and at least one second collagen layer; wherein said at least one second collagen layer (low density layer) has a density of up to about 90% of the first collagen layer (high density layer).

In some embodiments, the present invention provides a layered structure comprising at least two collagen layers each having a different density, wherein said at least two collagen layers comprise at least two first collagen layers and at least one second collagen layer; wherein said at least one second collagen layer (inner layer) is positioned between each of said at least two first collagen layers (external layers).

In some embodiments, the present invention provides a layered structure comprising at least two collagen layers each having a different density, wherein said at least two collagen layers comprise at least two first collagen layers and at least one second collagen layer; wherein said at least one second collagen layer (inner layer) is positioned between each of said at least two first collagen layers (external layers), and wherein said at least one second collagen layer (low density layer) has a density of up to about 90% of the at least two first collagen layers each independently (high density layers).

In some embodiments, the present invention provides a layered structure comprising at least two collagen layers each having a different density, wherein said at least two collagen layers comprise at least two first collagen layers and at least one second collagen layer; wherein said at least one second collagen layer (inner layer) is positioned between each of said at least two first collagen layers (external layers), and wherein said at least two first collagen layers (low density layer) have each independently a density of up to about 90% of the at least one second collagen layer (high density layers).

In some embodiments, said at least two collagen layers comprise at least one first collagen layer and at least one second collagen layer; wherein said at least one first collagen layer has a density of at least 90% higher than the density of said at least one second collagen layer.

In some further embodiments, said at least two first collagen layers have independently a density of at least 90% higher than the density of said at least one second collagen layer.

In some further embodiments, said at least one second collagen layer has a density of at least 90% higher than the density of each of said at least two first collagen layers.

According to some embodiments, the layered structure of the invention comprises two, three, four or five collagen layers. According to some embodiments, the layered structure of the invention may include multiple layers each with different densities.

The layered structure of the invention does not include defined separate layers; rather, it includes a gradient of densities, such that at least one section of the device has a different, and possibly varying, density than another section of the device. According to some embodiments, the density of the various sections ranges from 0.4-2 g/cm³. According to some embodiments, the density of the relatively highly dense collagen layer is in the range of about 1.0-1.7 g/cm³

According to some embodiments, the layered structure of the invention comprises three collagen layers, wherein the outer layers are highly dense, while the internal layer has a relatively low density. In some embodiments the internal layer may be a gel-like layer. According to some embodiments, the denser external layers have a different thickness than the internal layer. In some embodiments the thickness of the internal layer is higher than the thickness of the external layers.

According to some embodiments, the internal layer has a thickness of between about 0.5-5.0 mm. According to some embodiments, the internal layer has a thickness of between about 0.5-1.0 mm. According to some embodiments, the internal layer has a thickness of between about 1.0-2.0 mm. According to some embodiments, the internal layer has a thickness of between about 2.0-3.0mm. According to some embodiments, the internal layer has a thickness of between about 3.0-4.0 mm. According to some embodiments, the internal layer has a thickness of between about 4.0-5.0 mm.

According to some embodiments, the external layer has a thickness of between about 0.05-0.4 mm. According to some embodiments, the external layer has a thickness of between about 0.05-0.1 mm. According to some embodiments, the external layer has a thickness of between about 0.1-0.2 mm. According to some embodiments, the external layer has a thickness of between about 0.2-0.3 mm. According to some embodiments, the external layer has a thickness of between about 0.3-0.4 mm.

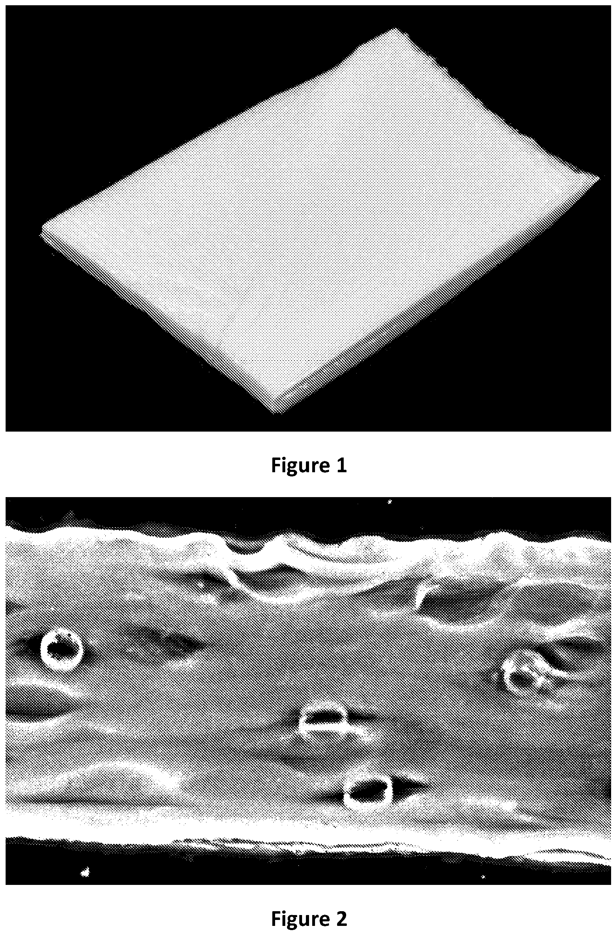

An embodiment of a layered structure of the invention is shown , e.g., in Figures 1 (photographic image with no magnification) and 2 (2X magnification of the cross section of the device). According to some embodiments, the average density of the external layers is about 1.41g/cm³. According to some embodiments, the average density of the internal layer is about 1.17g/cm³. According to some embodiments, the density of the external layer is in the range of about 1.0-1.7 g/cm³. According to some embodiments, the density of the internal layer is in the range of about 0.4-1.0 g/cm³.

Embodiments of the invention are directed to a method for preparing a layered structure of the invention, the method comprising: introducing collagen into a vessel; fibrillating the collagen to provide a collagen gel comprising collagen fibrils; compressing the collagen gel in the presence of distancing elements, so as to provide at least two layers having different densities; crosslinking the collagen gel; an drying the crosslinked collagen.

According to some embodiments, the collagen is fibrillated using a fibrillation agent. The collagen may be fibrillated by any method known in the art. According to some embodiments, the collagen is fibrillated by neutralizing its pH, e.g., by means of mixing it with a buffer solution having a neutral or basic pH. According to some embodiments, the fibrillation agents may include one or more bases and/or salts, such as sodium phosphate, Tris HCl, potassium hydroxide or sodium hydroxide.

Once the collagen is fibrillated, it may be at least partially compressed in the presence of distancing elements, so as to provide at least two layers having different densities. According to some embodiments, the compression of the collagen is conducted such that layers are formed. For example, when the collagen is compressed, the outer layers thereof are compressed more than the internal layers, wherein the collagen and particularly the internal layer/s are not fully compressed due to the presence of the distancing elements. Thus, the compression may be ceased at any appropriate state, for example, when relatively dense layers are formed on the outside of the device, while a relatively less dense layer is formed on the inside.

According to some embodiments, at least one of the external layers is compressed more than the internal layer. According to some embodiments the two external layers are compressed more than one internal layer, thereby providing a three-layered collagen entity, having two relatively high density external layers and one relatively low density internal layer. According to some embodiments the two external layers are compressed more than one internal layer, thereby providing a three-layered collagen entity, having one first external layer that is has relatively high density, a second external layer which has a lower density than the first external layer and one relatively low density internal layer. The compression provides a gradient of densities, ranging from high densities in the outer layers to lower densities in the more internal layers of the layered structure of the invention. The two external layers may be with similar density (about 10% difference) or with different densities, although both have higher densities than the internal layer.

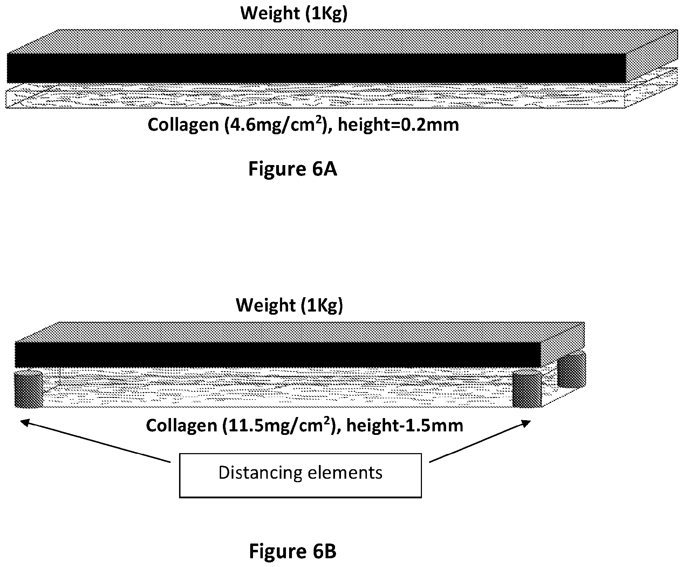

As mentioned above, the collagen is compressed in the presence of at least one distancing element that prevents the compressing element, e.g., a weight, from fully compressing the fibrillated collagen. Reference is made to Figures 6A and 6B, presenting the compression of fibrillated collagen without the presence of distancing elements (Fig. 6A) and in the presence of distancing elements (Fig. 6B). As shown, the distancing elements prevent the compressing element from fully compressing the fibrillated collagen, since they create a distance between the compressing element and the bottom of the reaction vessel. Accordingly, as presented, when compressing in the presence of distancing elements, the prepared multilayered layered structure of the invention is thicker than a layered structure of the inventionprepared without the use of distancing elements. It is noted that the final thickness of the multilayered layered structure of the invention is at least partially dependent on the height of the distancing elements.

According to some embodiments, the height of the distancing elements is about 0.1-10 mm. According to some embodiments, the height of the distancing elements is about 0.1-1.0mm. According to some embodiments, the height of the distancing elements is about 1.0-2.0mm. According to some embodiments, the height of the distancing elements is about 2.0-3.0mm. According to some embodiments, the height of the distancing elements is about 3.0-4.0mm. According to some embodiments, the height of the distancing elements is about 4.0-5.0mm. According to some embodiments, the height of the distancing elements is about 5.0-6.0mm. According to some embodiments, the height of the distancing elements is about 6.0-7.0mm. According to some embodiments, the height of the distancing elements is about 7.0-8.0mm. According to some embodiments, the height of the distancing elements is about 8.0-9.0mm. According to some embodiments, the height of the distancing elements is about 9.0-10mm.

According to some embodiments, the distancing elements are prepared from any appropriate material that is able to provide the desired distance, without interfering with the process for preparing the collagen, such as Teflon^{™}, stainless steel, silicone, polypropylene, polyethylene, or any combination thereof. According to some embodiments, the distancing elements are anchored into the reaction vessel. According to some embodiments, the distancing elements are vertically positioned in the reaction vessel. According to some embodiments, distancing elements may be added to the reaction vessel at any stage before compression. According to some embodiments, distancing elements may be added to the reaction vessel at any stage during compression, provided that the fibrillated collagen is not overly compressed before the distancing elements are added. According to other embodiments, the fibrillated collagen may be transferred to a reaction vessel containing distancing elements before the compression. Once the compression is concluded, the distancing elements may be separated from the prepared multilayered layered structure of the invention either by removing the distancing elements from the reaction vessel or by removing the multilayered layered structure of the invention from the reaction vessel.

Once at least partially compressed, the collagen may be crosslinked according to any known method in the art. According to some embodiments, the compressed collagen gel is contacted with a crosslinking agent.

According to some embodiments, the collagen is crosslinked by an enzymatic mediated process, by physical treatment (e.g., heat, UV radiation), or by means of a chemical cross-linking agent, wherein the crosslinking agent may comprise a reducing agent, such as, a reducing sugar or a reducing sugar derivative, or any combination thereof.

According to some embodiments, the crosslinking agent comprises an aldehyde or ketone mono sugar or mono sugar derivative, wherein the α-carbon is in an aldehyde or ketone state in an aqueous solution. According to some embodiments, the crosslinking agent includes compounds and reagents, as detailed in US 6, 346, 515. As detailed therein, the reducing sugars may form Schiff bases with the α or ε amino groups of the amino acids of the collagen molecule. The Schiff base may then undergo an Amadori Rearrangement to form a ketoamine product. Two adjacent ketoamine groups may then condense to form a stable intermolecular or intramolecular crosslink.

The reducing agents may include, for example, glycerose, threose, erythrose, lyxose, xylose, arabinose, ribose, allose, altrose, glucose, mannose, gulose, idose, galactose, talose, or any other diose, triose, tetrose, pentose, hexose, septose, octose, nanose or decose, or any combination thereof. For example, when the crosslinking agent comprises a ribose, a stable crosslink via a pentosidine group may be formed.

According to some embodiments, the degradation rate of the layered structure of the invention may be controlled by the extent of the crosslinking between the collagen fibrils. The extent of the cross linking may be controlled, e.g., by the concentration of the crosslinking agent, the temperature and the time during which the collagen fibrils are exposed to the crosslinking agent. According to some embodiments, the cross-linking may be performed at a cross-linker concentration range of about 0.01%-5%. According to some embodiments, the cross-linking may be performed at a temperature range of about 20-40°C. According to some embodiments, the cross-linking may be performed for a duration range of about 6-360 hours.

According to some embodiments, the tissue surrounding the layered structure of the invention is regenerated, at least partially, over time, such that it replaces the layered structure of the invention. Thus, the layered structure of the invention may provide space for the surrounding tissue to grow into. According to some embodiments, the distancing elements used in the process for preparing the layered structure of the invention provide a layered structure of the invention with voids between the collagen molecules, since the collagen was not fully compressed during the preparation of the device. The formed voids allow the surrounding tissue to penetrate into the formed spaces, to grow therein and over time, to replace the degrading collagen. According to some embodiments, the layered structure of the invention calcifies and subsequently ossifies, providing a base for bone cell population.

According to some embodiments, the layered structure of the invention is biodegradable. According to some embodiments, the layered structure of the invention is biodegraded within about 3-24 months. According to some embodiments, the layered structure of the invention is biodegraded within about 4-8 months. According to some embodiments, the layered structure of the invention is biodegraded within about 5-7 months. According to some embodiments, the layered structure of the invention is biodegraded within about 3-10 months. According to some embodiments, the layered structure of the invention is biodegraded within about 10-17 months. According to some embodiments, the layered structure of the invention is biodegraded within about 17-24 months. According to some embodiments, the layered structure of the invention is biodegraded within about six months. It is noted that the layered structure of the invention is considered biodegraded when more than about 70, 80, 90 or 95% is degraded.

According to some embodiments, the different layers of the layered structure of the invention may degrade at different rates, depending on their density. According to some embodiments, denser layers degrade at a slower rate than less dense layers.

According to some embodiments, once the collagen is crosslinked, the prepared layered structure of the invention is washed to remove residues of reactants, such as fibrillation agents, crosslinkers, non-cross-linked-collagen and the like. According to further embodiments, the collagen membrane is then at least partially dehydrated by any appropriate means, such as air drying, freeze-drying, critical point drying, or any combination thereof. The drying procedure may further sterilize the multilayered layered structure of the inventions and render them dry, effectively prolonging their shelf life. According to some embodiments, the layers in the dehydrated multilayered layered structure of the invention may be less apparent; however, according to some embodiments, rehydration with any appropriate hydrant, such as water, saline solution, blood, phosphate-buffered saline (PBS) solution, and the like, may restore the three dimensional structure of the multilayered layered structure of the invention. According to some embodiments, the multilayered layered structure of the invention is rehydrated *in vitro.* According to other embodiments, the multilayered layered structure of the invention is rehydrated *in vivo.*

According to some embodiments, the layered structure of the invention may comprise active agents having various therapeutic effects. According to some embodiments, the active agents are immobilized within the layered structure of the invention by the crosslinking agent, e.g., the reducing sugars, or by its natural tendency for binding to collagen. During the gradual biodegradation of the layered structure of the invention, such active agents may be gradually released into the body, thereby providing a controlled release system. Such active agents may include antimicrobial agents, anti-inflammatory agents, growth factors having tissue regeneration induction properties and the like, as well as any combination thereof.

Antimicrobial agents may include penicillin, cefalosporins, tetracyclines, streptomycin, gentamicin, sulfonamides, and miconazole. The anti-inflammatory agents may include cortisone, synthetic derivatives thereof, and the like. Tissue regeneration induction factors may include differentiation factors, bone morphogenetic proteins, attachment factor and growth factors, such as, fibroblast growth factors, platelet derived growth factors, transforming growth factors, cementum growth factors, insulin-like growth factors, and the like.

According to some embodiments, additional biodegradable materials may be added, such as polylactic acid (PLA), polyglycolide (PGA), chitosan, hyaluronic acid and mixtures thereof. According to further embodiments, any type of minerals, such as tri calcium phosphate, silicon oxide, hydroxyapatite may be added.

According to some embodiments, cells, such as human cells, bone cells, cell cultures, fibroblasts, chondrocytes, (mesenchymal) stem cells, osteocytes, adipocytes, endothelial cells may be seeded onto or into at least one of the layers, e.g., the external dense layer that is to be placed in the direction of the bone. Such a layer could act as a protective, semi-permeable layer, to provide the possibility of nutrition, e.g., fluid containing minerals and the like, supply to the inner layers of the device and/or to tissue found under the device, while protecting internal layers of the device and/or tissue under the device from direct interactions with other cell types. According to some embodiments, interaction of the internal layers and/or the tissue under the device is prevented due to the pore size of at least one layer in the multilayered layered structure of the invention, wherein the pore size is smaller than necessary for certain cell types to penetrate through.

According to some embodiments, the multilayered layered structure of the invention may be used in conjunction with a space-maintaining material ("space maintainer" or "spacer"). The term "in conjunction" is intended to cover uses in which the space maintainer is adjacent to the multilayered layered structure of the invention, is attached to the layered structure of the invention by any appropriate means, or is incorporated into the layered structure of the invention.

A space maintainer may be used in some procedures in order to maintain a space in which the regenerating cells can migrate and repopulate. In some instances, such a space occurs naturally, for example, when a tumor is excised from a bone. In other instances, such a space is not available, for example, in various types of periodontal or bone lesions. In such instances it may be necessary to insert filling material between the collagen membrane and the regenerating tissues. Examples of space maintainers are (i) hyaluronan (hyaluronic acid), (ii) mineralized freeze dried bone, (iii) deproteinazed bone, (iv) synthetic hydroxyapatite, (v) crystalline materials other than those mentioned under (ii)-(iv), enriched with osteocalcin or vitronectin, and (vi) heat-treated demineralized bone, wherein the bone-derived substances may be of human origin. Also possible are combinations of any of the above space maintainers, such as the combination of hyaluronan and with one or more of the other space maintainers.

For various applications depending on the size, form and location of the regenerating site, the space maintainers may be enriched with one or more of the antibacterial, anti-inflammatory and tissue-inductive factors mentioned above; and/or enriched with a substance intended to aid in maintaining the shape of the space maintainer matrix, e.g. one or more matrix proteins selected from the group comprising collagen, fibrin, fibronectin, osteonectin, osteopontin, tenascin, thrombospondin; and/or glycoseaminoglycans including heparin sulfate, dermatan sulfates, chondrointin sulfates, keratan sulfates, and the like.

According to some embodiments, the layered structure of the invention is designed such that it fills the space in which the tissue is to be regenerated. Accordingly, the use of space maintainer may not be necessary, since the tissue may be regenerated and replace the biodegrading layered structure of the invention. According to some embodiments, as detailed herein, the layered structure of the inventions may be prepared to have any size or shape.

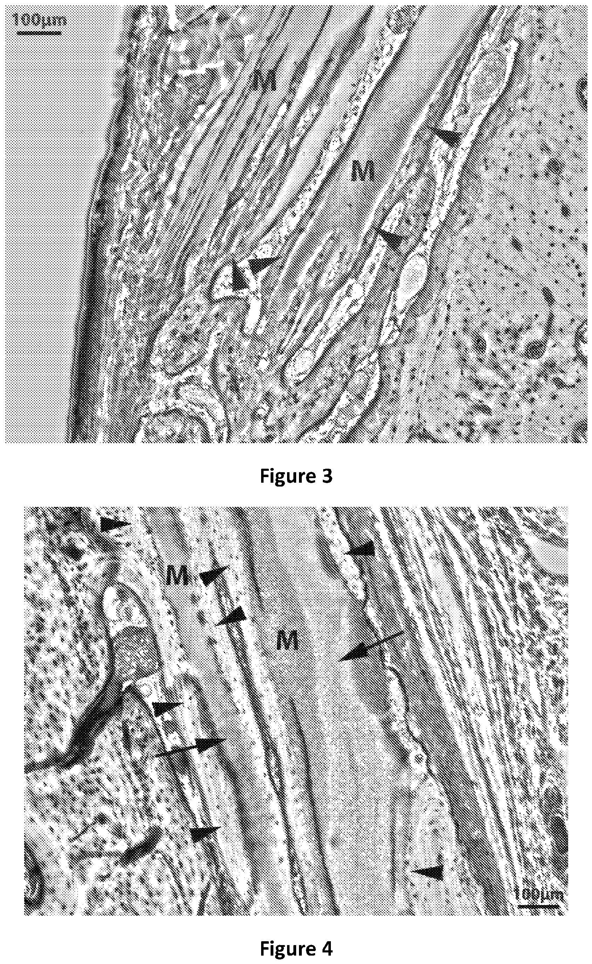

Reference is now made to Figures 3-5, presenting a photomicrograph of 4 weeks implantation in a dog L-shape bony defect. Note the initial ossification advancing in the middle layer of the device (arrows). The original collagen matrix of the device is marked (M) (Figure 3).
Figure 4 is a photomicrograph of 24 weeks implantation in a dog L-shape bony defect. Note the advanced ossification of the middle layer of the device (arrows).

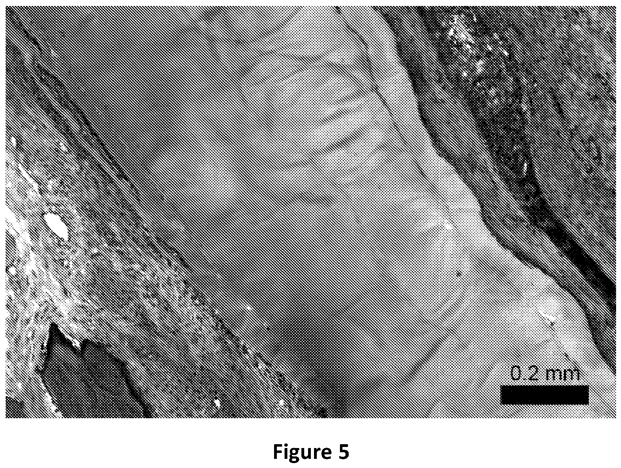

Figure 5 is a photomicrograph of 4 weeks implantation in a dog L-shape bony defect. Membrane with notable 3 layer structure surrounded by soft tissue (* low density; arrows: high density)

According to some embodiments, the multilayered layered structure of the invention comprises an internal scaffold, as presented in detail in US 62/317,569.

In order to better understand how the present invention may be carried out, the following examples are provided, demonstrating a process according to the present disclosure.

### EXAMPLES

### Example 1

An aliquot of 320 ml of collagen was mixed with 30 ml of fibrillation buffer composed of 200 mM Sodium Phosphate pH 11.2. After being mixed with the fibrillation buffer, the collagen was poured into a molding plate of 11 × 16 cm containing 3mm high Teflon-based distancing elements, to maintain the desired final thickness of the membrane. Fibrillation was allowed to proceed for 18 hours at 37°C. The resulted gel was compressed using 1 Kg weight for 20 hours at 37°C, wherein the distancing elements ensured that throughout the compression a distance of at least 3mm was maintained between the weight and the bottom of the molding plate. The collagen sheet was cross-linked in a 0.2 micron filtered medium of 10g ribose, 158g ethanol and 790g PBS for 11 days at 37°C. The resulting membrane was then washed with water, dehydrated with ethanol and dried by lyophilization. Figure 1 presents a low magnification of the prepared wet collagen membrane and Figure 2 presents a high magnification of a cross-section of the prepared wet collagen membrane.

## Claims

1. A layered structure comprising at least two collagen layers, wherein the layered structure includes a gradient of densities; and the layered structure is prepared by a method comprising:
introducing collagen into a vessel;
fibrillating the collagen to provide a collagen gel comprising collagen fibrils;
compressing the collagen gel in the presence of at least one distancing element,
so as to provide multiple collagen layers with a gradient of densities;
crosslinking the collagen gel; and
drying the crosslinked collagen.

2. The layered structure according to claim 1, wherein said at least two collagen layers comprise at least one first collagen layer and at least one second collagen layer; wherein said at least one second collagen layer (low density layer) has a density of up to about 90% of the first collagen layer (high density layer).

3. The layered structure according to claim 1, wherein said at least two collagen layers comprise at least two first collagen layers and at least one second collagen layer; wherein said at least one second collagen layer is positioned between each of said at least two first collagen layers.

4. The layered structure according to claim 3, wherein said at least one second collagen layer (low density layer) has a density of up to about 90% of the at least two first collagen layers each independently.

5. A method for preparing a layered structure comprising at least two collagen layers, said method comprising:
introducing collagen into a vessel;
fibrillating the collagen to provide a collagen gel comprising collagen fibrils;
compressing the collagen gel in the presence of at least one distancing element,
so as to provide multiple collagen layers with a gradient of densities;
crosslinking the collagen gel; and
drying the crosslinked collagen.

6. A surgical scaffold for use in soft tissue growth/augmentation comprising the layered structure of any one of claims 1-4.

7. The layered structure of any one of claims 1-4, for use in reinforcing soft tissues.

8. The layered structure of any one of claims 1-4, for use in treating bone growth/augmentation comprising guided bone regeneration (GBR) and guided tissue regeneration (GTR).

## Patentansprüche

1. Schichtstruktur, umfassend mindestens zwei Kollagenschichten, wobei die Schichtstruktur einen Dichtegradienten beinhaltet und die Schichtstruktur hergestellt ist durch ein Verfahren, umfassend:
Einführen von Kollagen in ein Gefäß;
Fibrillieren des Kollagens, um ein Kollagengel bereitzustellen, das Kollagenfibrillen umfasst;
Komprimieren des Kollagengels bei Anwesenheit von mindestens einem Abstandselement, sodass mehrere Kollagenschichten mit einem Dichtegradienten bereitgestellt werden;
Vernetzen des Kollagengels; und
Trocknen des vernetzten Kollagengels.

2. Schichtstruktur gemäß Anspruch 1, wobei die mindestens zwei Kollagenschichten mindestens eine erste Kollagenschicht und mindestens eine zweite Kollagenschicht umfassen, wobei die mindestens eine zweite Kollagenschicht (Schicht von geringer Dichte) eine Dichte von bis zu etwa 90 % der ersten Kollagenschicht (Schicht von hoher Dichte) aufweist.

3. Schichtstruktur gemäß Anspruch 1, wobei die mindestens zwei Kollagenschichten mindestens zwei erste Kollagenschichten und mindestens eine zweite Kollagenschicht umfassen, wobei die mindestens eine zweite Kollagenschicht jeweils zwischen den mindestens zwei Kollagenschichten positioniert ist.

4. Schichtstruktur gemäß Anspruch 3, wobei die mindestens eine zweite Kollagenschicht (Schicht von geringer Dichte) eine Dichte von bis zu etwa 90 % der mindestens zwei ersten Kollagenschichten jeweils unabhängig voneinander aufweist.

5. Verfahren zum Herstellen einer Schichtstruktur, umfassend mindestens zwei Kollagenschichten, das Verfahren umfassend:
Einführen von Kollagen in ein Gefäß;
Fibrillieren des Kollagens, um ein Kollagengel bereitzustellen, das Kollagenfibrillen umfasst;
Komprimieren des Kollagengels bei Anwesenheit von mindestens einem Abstandselement, sodass mehrere Kollagenschichten mit einem Dichtegradienten bereitgestellt werden;
Vernetzen des Kollagengels; und
Trocknen des vernetzten Kollagengels.

6. Chirurgisches Gerüst zur Verwendung zum/zur Weichgewebewachstum/- vermehrung, umfassend die Schichtstruktur nach einem der Ansprüche 1-4.

7. Schichtstruktur nach einem der Ansprüche 1-4 zur Verwendung zum Verstärken von Weichgewebe.

8. Schichtstruktur nach einem der Ansprüche 1-4 zur Verwendung zur Behandlung von Knochenwachstum/-vermehrung, umfassend geführte Knochenregeneration (GBR) und geführte Geweberegeneration (GTR).

## Revendications

1. Structure en couches comprenant au moins deux couches de collagène, dans laquelle la structure en couches comporte un gradient de densités ; et la structure en couches est préparée par un procédé comprenant :
l'introduction de collagène dans un récipient ;
la fibrillation du collagène pour obtenir un gel de collagène comprenant des fibrilles de collagène ;
la compression du gel de collagène en présence d'au moins un élément de distanciation, de manière à obtenir plusieurs couches de collagène avec un gradient de densités ;
la réticulation du gel de collagène ; et
le séchage du collagène réticulé.

2. Structure en couches selon la revendication 1, dans laquelle au moins ces deux couches de collagène comprennent au moins une première couche de collagène et au moins une deuxième couche de collagène ; dans laquelle au moins cette deuxième couche de collagène (couche de faible densité) a une densité allant jusqu'à environ 90 % de la première couche de collagène (couche de haute densité).

3. Structure en couches selon la revendication 1, dans laquelle au moins ces deux couches de collagène comprennent au moins deux premières couches de collagène et au moins une deuxième couche de collagène ; dans laquelle au moins cette deuxième couche de collagène est positionnée entre chacune de ces deux premières couches de collagène au moins.

4. Structure en couches selon la revendication 3, dans laquelle au moins cette deuxième couche de collagène (couche de faible densité) a une densité allant jusqu'à environ 90 % des deux premières couches de collagène au moins, chacune indépendamment.

5. Procédé pour préparer une structure en couches comprenant au moins deux couches de collagène, ce procédé comprenant :
l'introduction de collagène dans un récipient ;
la fibrillation du collagène pour obtenir un gel de collagène comprenant des fibrilles de collagène ;
la compression du gel de collagène en présence d'au moins un élément de distanciation, de manière à obtenir plusieurs couches de collagène avec un gradient de densités ;
la réticulation du gel de collagène ; et
le séchage du collagène réticulé.

6. Échafaudage chirurgical utilisé pour la croissance/l'augmentation des tissus mous comprenant la structure en couches de l'une quelconque des revendications 1-4.

7. Structure en couches de l'une quelconque des revendications 1-4, utilisée pour le renforcement des tissus mous.

8. Structure en couches de l'une quelconque des revendications 1-4, utilisée pour le traitement de la croissance/de l'augmentation osseuse comprenant la régénération osseuse guidée (ROG) et la régénération tissulaire guidée (RTG).
